(19) Europäisches Patentamt
European Patent Office
Office européen des brevets

(11) Veröffentlichungsnummer: 0 045 907
A1

(12) EUROPÄISCHE PATENTANMELDUNG

(21) Anmeldenummer: 81105997.1

(22) Anmeldetag: 30.07.81

(51) Int. Cl.³: C 07 D 207/456
A 01 N 43/36

(30) Priorität: 12.08.80 DE 3030481

(43) Veröffentlichungstag der Anmeldung:
17.02.82 Patentblatt 82/7

(84) Benannte Vertragsstaaten:
BE CH DE FR GB IT LI NL SE

(71) Anmelder: BAYER AG
Zentralbereich Patente, Marken und Lizenzen
D-5090 Leverkusen 1, Bayerwerk(DE)

(72) Erfinder: Bonse, Gerhard, Dr.
Wolfskaul 3
D-5000 Köln 80(DE)

(72) Erfinder: Blank, Heinz Ulrich, Dr.
Am Geusfelde 35
D-5068 Odenthal(DE)

(72) Erfinder: Paulus, Wilfried, Dr.
Deswatinesstrasse 90
D-4150 Krefeld 1(DE)

(72) Erfinder: Genth, Hermann, Dr.
Am Heckerhof 60
D-4150 Krefeld 1(DE)

(54) **Verwendung von N-alkyl-2,3-dichloro-maleinimiden als mikrobizides Mittel.**

(57) N-Alkyl-2,3-dichlor-maleinimide können als mikrobizides Mittel für technische Materialien verwendet werden.

EP 0 045 907 A1

BEZEICHNUNG GEÄNDERT
siehe Titelseite



BAYER AKTIENGESELLSCHAFT
5090 Leverkusen, Bayerwerk
Mn/bc/c
Zentralbereich
Patente, Marken und Lizenzen

## Mikrobizides Mittel

Die Erfindung betrifft mikrobizide Mittel, die N-Alkyl-2,3-dichlor-maleinimide enthalten.

Aus der US 2 865 730 ist bekannt, daß Halogen-Maleinimide herbizide Wirkung haben. In der GB 1 094 802 wird die Verwendung von Halogen-Maleinimiden als Wuchsstoffe beschrieben.

Es ist außerdem bekannt (DE-OS 2 156 967, US 3 734 927) daß N-(p-Fluorphenyl)-2,3-dichlor-maleinimid ein Wirkstoff gegen Citrusschorf ist. Aus Japan Kokai 73/39, 638 ist die Anwendung von N-(Fluorphenyl)-2,3-dichlor-maleinimiden zur Bekämpfung von Trichoderma viride in Pilzkulturen bekannt.

Es wurden neue mikrobizide Mittel gefunden, die N-Alkyl-2,3-dichlor-maleinimide der Formel

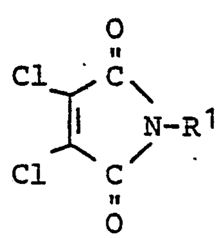

worin

$R^1$ für gegebenenfalls substituiertes Alkyl oder Cycloalkyl steht,

enthalten.

Die neuen N-alkyl- bzw.-cycloalkylsubstituierten Dichlormaleinimide haben überraschenderweise eine größere mikrobizide Wirkung als die entsprechenden N-phenylsubstituierten Verbindungen.

Alkyl ist bevorzugt ein geradkettiger oder verzweigter Kohlenwasserstoffrest mit 1 bis 16 Kohlenstoffatomen. Insbesondere bevorzugt ist ein Niederalkylrest mit bis zu etwa 6 Kohlenstoffatomen. Beispielsweise seien die folgenden Alkylreste genannt: Methyl, Ethyl, Propyl, Isopropyl, Butyl, Isobutyl, Pentyl, Isopentyl, Hexyl und Isohexyl.

Cycloalkyl ist bevorzugt ein cyclischer Kohlenwasserstoffrest mit 3 bis 10 Kohlenstoffatomen. Insbesondere bevorzugt sind der Cyclopentyl- und der Cyclohexylrest.

Als Substituenten des Restes $R^1$ seien in mikrobiziden Mitteln übliche Substituenten genannt. Beispielsweise können die folgenden Reste genannt werden: Halogen, wie Fluor, Chlor, Brom und Jod (bevorzugt Fluor und Chlor), Thiocyanat, $C_1$ bis $C_4$-Alkoxy, Cyano, $C_1$ bis $C_4$-Alkylthio und Phenyl.

Als bevorzugte mikrobizide Mittel der Formel (I) seien N-Alkyl-2,3-dichlor-maleinimide der Formel

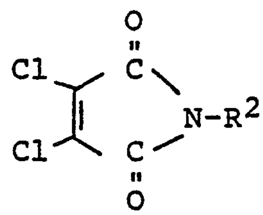

worin

$R^2$ für ein Niederalkyl-, Cyclopentyl- oder Cyclohexylrest steht,

genannt.

Die erfindungsgemäßen N-Alkyl-2,3-dichlor-maleinimide können in an sich bekannter Weise hergestellt werden (GB 818 192). Beispielsweise kann man Amine der Formel

$$H_2N-R^1$$

worin

$R^1$ die oben angegebene Bedeutung hat,

mit Dichlormaleinsäureanhydrid bei erhöhten Temperaturen, beispielsweise 70 bis 120°C, in Gegenwart von Säuren, beispielsweise Eisessig oder Salzsäure, umsetzen.

Die erfindungsgemäßen N-Alkyl-2,3-dichlor-maleinimide können bevorzugt als Wirkstoffe zur Bekämpfung von Mikroorganismen in technischen Materialien verwendet werden.

Technische Materialien, die durch die erfindungsgemäßen Wirkstoffe vor einer mikrobiellen Veränderung und Zerstörung geschützt werden sollen, sind beispielsweise

Klebstoffe, Leime, Papiere und Kartone, Textilien, Leder, Holz, Anstrichmittel, Putze, Emulsionen und Kunststoffartikel, die von Mikroorganismen befallen und/oder zersetzt werden können. Im Rahmen der zu schützenden Materialien seien auch Teile von Produktionsanlagen, beispielsweise Kühlwasser- und Kühlschmiermittel-Kreislaufe genannt, die durch Mikroorganismen beeinträchtigt werden können.

Mikroorganismen, die einen Abbau oder eine Veränderung der technischen Materialien bewirken können, sind beispielsweise Bakterien, Pilze, Hefen, Algen, Schleime und Viren. Vorzugsweise werden die erfindungsgemäßen N-Alkyl-2,3-dichlor-maleinimide gegen Pilze, insbesondere gegen holzverfärbende und holzzerstörende Pilze und Schimmelpilze, eingesetzt.

Als Mikroorganismen seien beispielsweise die folgenden Gattungen genannt:

Alternaria, wie Alternaria tenuis,
Aspergillus, wie Aspergillus niger,
Chaetomium, wie Chaetomium globosum,
Coniophora, wie Coniophora cerebella,
Lentinus, wie Lentinus tigrinus,
Penicillium, wie Penicillium glaucum,
Polyporus, wie Polyporus versicolor,
Pullularia wie Pullularia pullulans,
Sclerophoma, wie Sclerophoma pityophila,
Trichoderma, wie Trichoderma viride,
Staphylococcus, wie Staphylococcus aureus

Je nach ihrem Anwendungsgebiet können die erfindungsgemäßen N-Alkyl-2,3-dichlor-maleinimide in die üblichen Formulierungen überführt werden, wie Lösungen, Emulsionen, Suspensionen, Pulver, Pasten und Granulate. Diese können in an sich bekannter Weise hergestellt werden, z.B. durch Vermischen der Wirkstoffe mit einem Streckmittel aus flüssigem Lösungsmittel und/oder festen Trägerstoffen, gegebenenfalls unter Verwendung von oberflächenaktiven Mitteln, wie Emulgatoren und/oder Dispergiermitteln, wobei beispielsweise im Falle der Benutzung von Wasserstreckmitteln, gegebenenfalls organische Lösungsmittel, als Hilfsmittel verwendet werden können.

Flüssige Lösungsmittel der Wirkstoffe können z.B. Wasser, Alkohol, beispielsweise niederer aliphatischer Alkohol, vorzugsweise Ethanol und Isopropanol, und aromatische Alkohole, wie Benzylalkohol, flüssige Kohlenwasserstoffe, wie Benzinfraktionen, chlorierte Kohlenwasserstoffe, wie 1,2-Dichlorethan sein.

Feste Trägerstoffe, die bei der Herstellung der fertigen Anwendungsformen des Wirkstoffes zugegeben werden können, können beispielsweise Talkum, Kieselgur, Bentonit, Kaolin, Diatomeenerde und synthetische Gesteinsmehle sein.

Oberflächenaktive Mittel können handelsübliche Emulgatoren, wie Alkylsulfonat, Alkylarylsulfonate, Arylsulfonate, Alkylamidsulfonate, Alkylarylpolyether-

alkohole, Polyoxyethylen-Fettsäureester oder Dispergiermittel, wie Lignin, Methylcellulose, Polyoxyethylen-polyoxypropylenblockpolymere, sein.

Die Anwendungsform des erfindungsgemäßen mikrobiziden Mittels enthält im allgemeinen 0,1 bis 95 Gew.-%, bevorzugt 0,5 bis 90 Gew.-%, des erfindungsgemäßen N-Alkyl-2,3-dichlor-maleinimids als Wirkstoff.

Die für den Schutz technischer Materialien erforderlichen Wirkstoffmengen können in größeren Bereichen variiert werden. Im allgemeinen liegen sie im Bereich von 0,0001 bis 5 Gew.-%, vorzugsweise im Bereich von 0,01 bis 2 Gew.-%, bezogen auf die Gesamtmenge des zu schützenden Materials.

Die erfindungsgemäßen Wirkstoffe können in den Formulierungen in Mischung mit anderen bekannten Wirkstoffen vorliegen. Beispielsweise seien die folgenden Wirkstoffe genannt: Benzimidazolyl-methylcarbamat, Tetramethyl-thiuramdisulfid, N-Fluordichlormethylthiophthalimid und N,N'-Dimethyl-N'-phenyl-(N-fluordichlormethylthio)-sulfamid.

A) Herstellungsbeispiel

Zu einer Lösung von 334 g (2,0 Mol) Dichlormaleinsäureanhydrid in 1400 ml Eisessig werden 146 g (2,0 Mol) n-Butylamin, gelöst in 300 ml Eisessig, zugefügt. Nach zweistündigem Rühren bei 80°C läßt man abkühlen und versetzt die Reaktionsmischung mit Eiswasser. Die ausgefallenen gelblichen Kristalle werden abgesaugt, mit Wasser gewaschen und getrocknet.
Ausbeute: 390,0 g (88 % der Theorie) vom Schmp. 37°C.

B) Bestimmung der minimalen Hemmkonzentration (MHK):

Ein Agar, der aus Bierwürze und Pepton hergestellt wird, wird mit dem Wirkstoff in Konzentrationen von 0,1 mg/l bis 2000 mg/l versetzt. Nach Erstarren des Agars erfolgt die Kontamination mit Reinkulturen der in der Tabelle 1) aufgeführten Testorganismen. Nach zweiwöchiger Lagerung bei 28°C und 60 bis 70 % relativer Luftfeuchtigkeit wird die MHK bestimmt. MHK ist die niedrigste Konzentration an Wirkstoff, bei der das Wachstum der angegebenen Mikrobenart gehemmt wird; sie ist in der nachstehenden Tabelle 1) angegeben.

Tabelle 1

MHK-Werte in mg/l

| Testorganismen | 1. N-Butyl-2,3-dichlor-maleinimid | 2. N-iso-Butyl-2,3-dichlor-maleinimid | 3. N-Pentyl-2,3-dichlor-maleinimid | 4. N-2-Dimethyl-propyl-2,3-dichlor-maleinimid | 5. N-Hexyl-2,3-dichlor-maleinimid | 6. N-2-Ethyl-hexyl-2,3-dichlor-maleinimid | 7. N-Cyclo-hexyl-2,3-dichlor-maleinimid | N-p-Fluor-phenyl-2,3-dichlor-maleinimid (Vergleich) |
|---|---|---|---|---|---|---|---|---|
| Alternaria tenuis | 50,0 | 20,0 | 20,0 | 35,0 | 20,0 | 100,0 | 35,0 | 150,0 |
| Aspergillus niger | 50,0 | 100,0 | 50,0 | 100,0 | 50,0 | 50,0 | 100,0 | 150,0 |
| Chaetomium globosum | 150,0 | 100,0 | 50,0 | 100,0 | 50,0 | 50,0 | 100,0 | 200,0 |
| Coniophora cerebella | 3,5 | 1,5 | 1,0 | 1,5 | 2,0 | 0,5 | 0,5 | 2,0 |
| Lentinus tigrinus | 20,0 | 5,0 | 10,0 | 5,0 | 10,0 | 35,0 | 3,5 | 35,0 |
| Penicillium glaucum | 50,0 | 50,0 | 50,0 | 10,0 | 10,0 | 50,0 | 50,0 | 150,0 |
| Polyporus versicolor | 100,0 | 100,0 | 50,0 | 50,0 | 50,0 | 100,0 | 100,0 | 1000,0 |
| Pullularia pullulans | 10,0 | 15,0 | 7,5 | 10,0 | 10,0 | 10,0 | 7,5 | 75,0 |
| Sclerophoma pityophila | 15,0 | 3,5 | 3,5 | 1,5 | 3,5 | 0,1 | 3,5 | 15,0 |
| Trichoderma viride | 100,0 | 500,0 | 200,0 | 200,0 | 150,0 | 350,0 | 200,0 | >2000,0 |

Patentansprüche

1) Mikrobizides Mittel, enthaltend N-Alkyl-2,3-dichlor-maleinimide der Formel

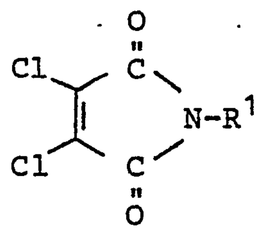

worin

$R^1$ für gegebenenfalls substituiertes Alkyl oder Cycloalkyl steht.

2) Mikrobizide Mittel nach Anspruch 1, enthaltend N-Alkyl-2,3-dichlor-maleinimide der Formel

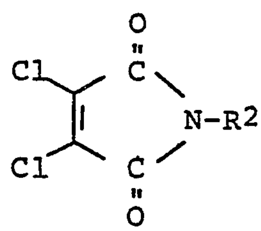

worin

$R^2$ für einen Niederalkyl- oder Cyclohexylrest steht.

3) Verwendung von mikrobiziden Mitteln nach Anspruch 1 zum Schutz technischer Materialien.

4) Mikrobizide Mittel nach den Ansprüchen 1 und 2, enthaltend 0,1 bis 55 Gew.-% N-Alkyl-2,3-dichlor-maleinimid.

0045907

Europäisches Patentamt

# EUROPÄISCHER RECHERCHENBERICHT

Nummer der Anmeldung
EP 81 10 5997

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 3 890 270 (P.P. MINIERI)<br>* Zusammenfassung *<br>--- | 1-4 |
| | DE - B - 1 108 224 (I.C.I.)<br>* Insgesamt *<br>--- | 1-4 |
| | DE - B - 1 146 694 (BASF)<br>* Insgesamt *<br>--- | 1-4 |
| D | US - A - 3 734 927 (S. KAWADA)<br>* Zusammenfassung *<br>--- | 1-4 |
| D | US - A - 2 865 730 (R.L. GATES)<br>* Seite I; Spalte I *<br>--------- | 1-4 |

KLASSIFIKATION DER ANMELDUNG (Int. Cl.3)

C 07 D 207/456
A 01 N 43/36

RECHERCHIERTE SACHGEBIETE (Int. Cl.3)

C 07 D 207/456
A 01 N 43/36

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 16. September 1981 | MAISONNEUVE |

EPA form 1503.1 06.78